# EUROPÄISCHE PATENTANMELDUNG

(11) **EP 3 159 002 A1**
(43) Veröffentlichungstag der Anmeldung: **26.04.2017**
(21) Anmeldenummer: 15191070.0
(22) Anmeldetag: 22.10.2015
(51) Int. Cl.: A61K 36/30

(54) **VERFAHREN ZUR HERSTELLUNG VON SPEZIAL-EXTRAKTEN AUS SYMPHYTUM**

(71) Anmelder: Merck Patent GmbH, 64293 Darmstadt (DE)
(72) Erfinder: Merck Patent GmbH, 64293 Darmstadt (DE)
(74) Vertreter: Frei Patent Attorneys

(57) **Zusammenfassung**

Ein Verfahren zur Herstellung eines Spezial-Extrakts aus Symphytum weist mindestens einen, insbesondere alle, der folgenden Schritte auf:
- Bereitstellen eines Suspensionssystems aus Symphytum;
- differentielles Fällen von Schleimpolysacchariden in dem Suspensionssystem, wobei die differentielle Fällung die folgenden Schritte aufweist:
- erste differentielle Schleimfällung von hochmolekularen Schleimpolysacchariden als erster Niederschlag in dem Suspensionssystem, Abtrennung des ersten Niederschlags und eines Extraktionsrückstands als Raffinat und Gewinnung einer ersten Extraktphase;
- zweite differentielle Schleimfällung von niedermolekularen Schleimpolysacchariden als zweiter Niederschlag aus der ersten Extraktphase, Abtrennung des zweiten Niederschlags und Gewinnung einer zweiten Extraktphase;

- selektive Entfernung von Pyrrolizidin-Alkaloiden aus dem zweiten Niederschlag und/oder der zweiten Extraktphase; und
- Vereinigen des zweiten Niederschlags, insbesondere des Pyrrolizidin-Alkaloidegereinigten zweiten Niederschlags, und der zweiten Extraktphase, insbesondere der Pyrrolizidin-Alkaloide-gereinigten zweiten Extraktphase, zum Spezial-Extrakt.

## Beschreibung

Die Erfindung bezieht sich auf das Gebiet der Herstellung von Spezial-Extrakten. Sie bezieht sich insbesondere auf ein Verfahren zur Herstellung eines Spezial-Extrakts aus Symphytum, ein Spezial-Extrakt aus Symphytum sowie ein Verfahren zur Herstellung einer ersten, schleimfreien und/oder einer zweiten, schleimhaltigen Extraktionsphase, und ein Verfahren zum Fällen von Schleimpolysacchariden in einem Suspensionssystems aus Symphytum sowie ein Verfahren zur selektiven Entfernung von Pyrrolizidin-Alkaloiden aus einer Extraktphase aus Symphytum und/oder einem von einer Extraktphase getrennten Niederschlag aus Symphytum gemäss dem Oberbegriff der entsprechenden unabhängigen Patentansprüche.

Symphytum, auch Beinwell genannt, ist eine traditionelle Arzneipflanze, deren Wirkung bereits in der Antike bekannt war. Die antiinflammatorischen, analgetischen und geweberegenerierenden Eigenschaften werden sowohl dem Beinwellkraut, bestehend aus den frischen und/oder getrockneten oberirdischen Teilen, als auch der Beinwellwurzel, bestehend aus frischen und/oder getrockneten unterirdischen Teilen, zugeschrieben. In jüngster Vergangenheit hat sich der Indikationsbereich von Beinwellwurzelextrakten durch den Nachweis der perkutanen Wirksamkeit bei Sprunggelenksdistorsion bestätigt. Dabei erwies sich Beinwell in Bezug auf den Rückgang von Schmerz, Entzündung, Schwellung, Bewegungseinschränkung und globale Wirksamkeit als klinisch und signifikant überlegen. Darüber hinaus haben moderne Testverfahren und Anwendungsbeobachtungen die Wirksamkeit und Unbedenklichkeit bei unterschiedlichen Muskel- und Gelenkbeschwerden bestätigt. Somit hat sich die medizinische Verwendung von Präparaten aus allen, insbesondere den unterirdischen, Teilen der Pflanze (Symphytum) gut etabliert und die Präparate werden neben der Behandlung von Prellungen, Zerrungen oder Verstauchungen auch bei schmerzhafter Gelenkarthrose und akuten Rückenschmerzen angewendet.

Die Bestandteile der Beinwellpflanze enthalten 0.6-4.7% Allantoin, reichlich Schleimpolysaccharide (25-30%) aufweisend Fructose- und Glucose-Einheiten, Phenolsäuren wie Rosmarinsäure (bis 0.2%), Chlorogensäure (0.012%) sowie Kaffeesäure (0.004%) und alpha-Hydroxykaffeesäure, Glycopeptide, Aminosäuren und Triterpensaponine in Form von monodesmosidischen und bidesmosidischen Glycosiden.

Als Wirkstoffe, also klinisch wirksame Bestandteile der Symphytum, kommen Allantoin, ein mit Harnsäure verwandtes Purinderivat, die Schleimpolysaccharide, Gerbstoffe und Triterpensaponine in Frage. Für die Pharmakodynamik sind Rosmarinsäure und andere Hydroxyzimtsäurenderivate von zentraler Bedeutung. Vermutlich sind alle Wirkstoffgruppen von Relevanz, die genauen Wirkmechanismen sind jedoch noch nicht vollständig geklärt.

Ebenfalls enthalten alle Pflanzenteile von Symphytum Pyrrolizidin-Alkaloide in Form ihrer N-Oxide. Symphytum weist etwa 150 verschiedene Pyrrolizidin-Alkaloide auf, die wichtigsten sind 7-Acetyllycopsamine, 7-Acetylintermedine zusammen mit geringen Mengen an Intermedine, Lycopsamin und Symphytine. Die Gesamtmenge von Pyrrolizidin-Alkaloiden variiert von 0.013 % bis 1.2 %, auch aus methodenspezifischen Gründen (Analyse). Die Pyrrolizidin-Alkaloide Echimidine und Symlandine werden in Symphytum nicht gefunden. Bei den Pyrrolizidin-Alkaloiden handelt es sich möglicherweise um mutagene bzw. carcinogene Substanzen, weshalb auf die innerliche Anwendung der Droge, sowie daraus hergestellte Zubereitungen, gänzlich verzichtet wird. Die sehr beliebte äusserliche Anwendung bei intakter Haut erscheint jedoch vertretbar, weshalb in Europa derzeit behördlicherseits definierte Grenzwerte für Tagesdosen von maximal 0.35 Mikrogramm Pyrrolizidin-Alkaloiden toleriert werden. In jedem Fall sollte es aus toxikologischer Sicht und aus Gründen der Vorsorge wünschenswert sein, den Gehalt an Pyrrolizidin-Alkaloiden möglichst gering zu halten.

Im industriellen Bereich ist bisher ein Verfahren bekannt, welches darauf beruht, dass ein alkoholischer Extrakt aus Petasites hybridus zur Abreicherung der Pyrrolizidin-Alkaloide mit einem Kationenaustauscher behandelt wird. Auf diese Weise können in diesen Extrakten mehr als 93% der Pyrrolizidin-Alkaloide entfernt werden (siehe Ch. Mauz et. al. in Pharmaceutica Acta Helvetiae, Band 60 (1985), 256-259). Petasites weist im Gegensatz zu Symphytum keine Schleimpolysaccharide auf.

Das hohe durch entsprechende Studien erkannte Wirkpotential der Beinwellpflanze rechtfertigt die Optimierung einer technologisch-methodischen Extrakt-Formulierung, weshalb vorliegender Erfindung daher mindestens die Aufgabe zugrunde liegt, ein Verfahren zur Herstellung von Spezial-Extrakten aus Symphytum bereitzustellen, welches durch eine im Wesentlichen produktschonende Verfahrenstechnologie eine hohe Ausbeute an pharmazeutisch relevanten Inhaltsstoffen ermöglicht.

Es ist deshalb Aufgabe der Erfindung, ein Verfahren zur Herstellung eines Spezial-Extrakts aus Symphytum, das Spezial-Extrakt aus Symphytum sowie ein Verfahren zur Herstellung einer ersten, schleimfreien und/oder einer zweiten, schleimhaltigen Extraktionsphase, und ein Verfahren zum Fällen von Schleimpolysacchariden in einem Suspensionssystems aus Symphytum sowie ein Verfahren zur selektiven Entfernung von Pyrrolizidin-Alkaloiden aus einer Extraktphase und/oder einem von einer Extraktphase getrennten Niederschlag bereitzustellen, welche eine optimierte Herstellung des Spezial-Extrakts ermöglichen.

Diese Aufgabe bzw. einen wesentlichen Teil dieser Aufgabe löst ein Verfahren zur Herstellung eines Spezial-Extrakts aus Symphytum, sowie Verfahren zur Herstellung einer ersten, schleimfreien Extraktionsphase aus Symphytum, ein Verfahren zur Herstellung einer zweiten, schleimhaltigen Extraktionsphase aus einer ersten schleimfreien Extraktionsphase, ein Verfahren zum Fällen von Schleimpolysacchariden in einem schleimigen Suspensionssystem aus Symphytum, ein Verfahren zur selektiven Entfernung von Pyrrolizidin-Alkaloiden aus einem gefällten und von einer Extraktphase getrennten Niederschlags aus Symphytum, ein Verfahren zur selektiven Entfernung von Pyrrolizidin-Alkaloiden aus einer Extraktphase aus Symphytum, und ein Spezial-Extrakt aus Symphytum mit den Merkmalen der entsprechenden unabhängigen Patentansprüche.

Das Verfahren zur Herstellung eines Spezial-Extrakts aus Symphytum weist mindestens einen, insbesondere alle, der folgenden Schritte auf:
- Bereitstellen eines Suspensionssystems aus Symphytum;
- differentielles Fällen von Schleimpolysacchariden in dem Suspensionssystem, wobei die differentielle Fällung die folgenden Schritte aufweist:
   - erste differentielle Schleimfällung von hochmolekularen Schleimpolysacchariden als erster Niederschlag in dem Suspensionssystem, Abtrennung des ersten Niederschlags und eines Extraktionsrückstands als Raffinat und Gewinnung einer ersten Extraktphase;
   - zweite differentielle Schleimfällung von niedermolekularen Schleimpolysacchariden als zweiter Niederschlag aus der ersten Extraktphase, Abtrennung des zweiten Niederschlags und Gewinnung einer zweiten Extraktphase;
- selektive Entfernung von Pyrrolizidin-Alkaloiden aus dem zweiten Niederschlag und/oder der zweiten Extraktphase; und
- Vereinigen des zweiten Niederschlags, insbesondere des Pyrrolizidin-Alkaloide-gereinigten zweiten Niederschlags, und der zweiten Extraktphase, insbesondere der Pyrrolizidin-Alkaloid-gereinigten zweiten Extraktphase, zum Spezial-Extrakt.

Ein Suspensionssystem aus Symphytum kann schleimig sein und dadurch viskos sein, da Symphytum Schleimstoffe, die sogenannten Schleimpolysaccharide, aufweist. Viskos bedeutet in diesem Zusammenhang höher viskos als Wasser bei Raumtemperatur. Die Schleimpolysaccharide lösen sich kolloidal im Suspensionssystem bzw. werden als Bestandteil von Extraktivstoffen im Suspensionssystem aufgeschlossen bzw. gequollen. In anderen Ausführungsformen ist es möglich, dass anstelle eines Suspensionssystems eine Extraktlösung aus Symphytum bereitgestellt wird. Eine solche Extraktlösung könnte durch Mazerieren von Pflanzenteilen in einem Lösungsmittel, insbesondere einem alkoholischen Lösungsmittel, insbesondere einem wässrig-alkoholischen Lösungsmittel, bereitgestellt werden. Bei der Herstellung einer solchen Extraktlösung liegt das Extraktionssystem nicht zwingend in suspendierter Form vor, daher kann die nachfolgende Beschreibung der Erfindung auch auf nach anderen Extraktionsverfahren hergestellten Extraktionssysteme anstelle des Suspensionssystems gelesen werden. In einem solchen Fall muss die Leseart der Beschreibung entsprechend angepasst werden.

Es sei angemerkt, dass Symphytum ein Oberbegriff für alle Symphytum Unterarten ist. Symphytum kann beispielsweise Symphytum officinale L. aufweisen. Die hier beschriebenen Verfahren lassen sich nicht nur auf eine spezielle Unterart von Symphytum, wie beispielsweise Symphytum officinale L., anwenden sondern auf alle Symphytum-Arten.

Spezial-Extrakte, beispielsweise aus Pflanzen, unterscheiden sich von herkömmlichen Extrakten dadurch, dass bei der Herstellung neben dem reinen Extrahieren noch weitere Bearbeitungsschritte vorgenommen werden. Bei der Herstellung eines Spezial-Extrakts handelt es sich um einen komplexen, vielstufigen Extraktions- und Reinigungsprozess. Dabei können unerwünschte Inhaltsstoffe, wie beispielsweise Pyrrolizidin-Alkaloide, entfernt und die erwünschten Wirkstoffe, welche die Wirksamkeit des Spezial-Extrakts bestimmen, angereichert werden.

Der Extraktionsrückstand ist als feste Restphase beziehungsweise Raffinat zu verstehen, er weist beispielsweise die festen Pflanzenteile, auf, welche nicht in der Extraktphase gelöst sind.

In anderen Ausführungsformen ist es möglich, dass der Extraktionsrückstand vor dem Fällen der Schleimpolysaccharide von dem Suspensionssystem abgetrennt wird. In diesem Fall, wird bei der ersten differentiellen Schleimfällung der erste Niederschlag von der ersten Extraktphase abgetrennt, da der Extraktionsrückstand bereits als Raffinat von dem Suspensionssystem abgetrennt wurde.

Die Anwendung von Spezial-Extrakten hat mehrere Vorteile. So kann die Wirkstoffkonzentration im Spezial-Extrakt erhöht werden. Unerwünschte Nebenprodukte werden bei der Extraktion oder weiteren Bearbeitungsschritten entfernt und das Spezial-Extrakt kann dadurch besser verträglich werden. Des Weiteren kann die Zusammensetzung und die Menge der Inhaltsstoffe gegebenenfalls standardisiert und/oder quantifiziert werden. Dies garantiert eine gleichbleibende Qualität des Spezial-Extrakts.

Bei dem Verfahren zur Herstellung eines Spezial-Extrakts aus Symphytum können hochmolekulare Schleimpolysaccharide als Niederschlag in dem schleimigen Suspensionssystem gefällt und nach dem Fällen von einer Extraktphase (flüssige Phase) abgetrennt werden. Die Extraktphase kann auch als Primärextrakt bezeichnet werden. Der Niederschlag und das abgetrennte Primärextrakt bzw. der abgetrennten Extraktphase können dabei einzeln von Pflanzenstoffen mit gesundheitsschädlichem Potenzial, wie beispielsweise Pyrrolizidin-Alkaloiden, gereinigt werden und nach der Pyrrolizidin-Alkaloid-Reinigung wieder vereinigt bzw. zusammengefügt werden. Dadurch wird es möglich, dass das Spezial-Extrakt nur einen geringen Pyrrolizidin-Alkaloid-Gehalt aufweist, wodurch die Anwendung des Spezial-Extrakts im therapeutischen Bereich ermöglicht werden kann.

Das Fällen von kolloidal gelösten Schleimpolysacchariden im Suspensionssystem aus Symphytum als Niederschlag kann eine differentielle Schleimfällung aufweisen. Auf diese Weise können differentiell bzw. schrittweise spezifische Extraktivstoffe, wie unerwünschte und/oder wirksame Schleimpolysaccharide, aus dem Suspensionssystem gefällt und gegebenenfalls als unlösliche Aggregate von der Extraktphase abgetrennt werden.

Das Spezial-Extrakt kann einen Pyrrolizidin-Alkaloid-Gehalt von maximal 0.5 ppm, insbesondere maximal 0.1 ppm, aufweisen. Dadurch wird es möglich, dass das Spezial-Extrakt aus Symphytum ohne Bedenken zu einer Creme und/oder Salbe weiterverarbeitet werden kann, die zur therapeutischen Behandlung, insbesondere zur therapeutischen Behandlung von Schmerzen, Entzündungen, Schwellungen, Bewegungseinschränkungen, Prellungen, Zerrungen, Vertauchungen, Gelenkarthrose, Rückenschmerzen, Muskelbeschwerden und/oder Gelenkbeschwerden verwendet werden kann.

Das Suspensionssystem, in welchem die Schleimpolysaccharide kolloidal gelöst sind, kann durch eine diskontinuierliche Extraktion hergestellt und/oder bereitgestellt werden. Dadurch wird es möglich unterschiedliche Extraktivstoffe effektiv während den einzelnen diskontinuierlichen Extraktionsschritten aus der Pflanze Symphytum zu extrahieren.

Die diskontinuierliche Extraktion kann dabei eine polaritätsgesteuerte diskontinuierliche Extraktion aufweisen, wobei die diskontinuierliche Extraktion durch das mehrstufige Einstellen der Polarität der Extraktionsphase erreicht bzw. ermöglicht werden kann. Die diskontinuierliche oder auch mehrstufige Extraktion kann im vorliegenden Fall auch als fraktionierte Extraktion bezeichnet werden.

Die diskontinuierliche Extraktion kann die folgenden Schritte aufweisen:
- Bereitstellen einer suspendierten, ersten, im Wesentlichen schleimfreien Extraktionsphase aus Symphytum; und
- Herstellung einer zweiten, schleimhaltigen Extraktionsphase.

In der ersten Extraktionsphase sind die Schleimpolysaccharide noch nicht kolloidal gelöst, also noch nicht aufgeschlossen bzw. gequollen. Daher ist die erste Extraktionsphase niederviskos und durch die Schleimpolysaccharide unbeeinflusst. Dadurch wird es möglich, dass in der im Wesentlichen schleimfreien, ersten Extraktionsphase die Extraktivstoffe, wie bspw. die Wirkstoffe, effizient freigesetzt und einfach gelöst werden können. Die Anwesenheit von Schleimstoffen, wie beispielsweise Schleimpolysacchariden, erschwert die Diffusionsvorgänge und damit die Extraktion von Wirkstoffen aus Symphytum. Des Weiteren können die Schleimpolysaccharide in einem zweiten Schritt bei der Herstellung der schleimhaltigen, zweiten Extraktionsphase aus Symphytum kolloidal gelöst und aufgeschlossen werden und können somit für das Spezial-Extrakt besonders gut und einfach zugänglich gemacht werden.

Die erste, im Wesentlichen schleimfreie Extraktionsphase kann durch ein Verfahren, aufweisend mindestens einen der folgenden Schritte:
- Bereitstellen von frischen, gefrorenen und/oder getrockneten Pflanzenteilen der Symphytum;
- Versetzen der Pflanzenteile mit einem niedermolekularem Alkohol, insbesondere mit Ethanol;
- Einstellen des Alkoholgehaltes der ersten Extraktionsphase (1) auf 65-75 Vol%, insbesondere auf 70 Vol%.
hergestellt bzw. bereitgestellt werden.

Niedermolekulare Alkohol wird als Alkohol verstanden der maximal zehn C-Atome aufweist, beispielsweise Ethanol, Propanol, Isopropanol usw..

Das Verfahren zur Herstellung einer im Wesentlichen schleimfreien, ersten Extraktionsphase als solches ist mit dem Verfahren zur Herstellung eines Spezial-Extrakts durch eine gemeinsame erfinderische Idee, nämlich die Verbesserung bzw. Vereinfachung der Herstellung eines Spezial-Extrakts aus Symphytum, verbunden. Aus diesem Grund kann das Verfahren zur Herstellung der ersten, im Wesentlichen schleimfreien Extraktionsphase aus Symphytum auch als unabhängiges Verfahren oder im Zusammenhang mit dem Verfahren zur Herstellung des Spezial-Extrakts oder individuell im Zusammenhang mit einem der andern im diesem Text beschriebenen Verfahren betrachtet werden.

Die bereitgestellten frischen, gefrorenen und/oder getrockneten Pflanzenteile der Symphytum können oberirdische und/oder unterirdische Pflanzenteile der Symphytum aufweisen. Die gefrorenen Planzenteile können dabei auch tiefgefroren sein. Die Verwendung von tiefgefrorenen Pflanzenteile hat zum Vorteil, dass enzymatische Zersetzungsprozesse in den Pflanzenteilen beispielsweise nach der Ernte unterbunden bzw. minimiert werden können. Dadurch können biochemische Prozesse der Nacherntephysiologie und Verderb unterbunden werden, was somit eine optimale Konservierung allfälliger Wirkstoffe sicherstellt.

In Ausführungsformen der Erfindung werden die Pflanzenteile in frischer, gefrorener, tiefgefrorener und/oder getrockneter Form zu einer Grösse von 0.5 cm bis 2.5 cm vorzerkleinert bereitgestellt.

In Ausführungsformen der Erfindung werden die vorzerkleinerten Pflanzenteile durch eine Nasszerkleinerung und/oder Turboextraktion weiter zerkleinert, wobei die Pflanzenteile auf eine Grösse von 0.1 mm bis 1 mm zerkleinert werden können. Auf diese Weise entsteht ein Suspensionssystem aus den fein zerkleinerten Pflanzenteilen und einem Lösungsmittel, das für die Nasszerkleinerung und/oder der Turboextraktion zu den vorzerkleinerten Pflanzenteilen zugegeben wird. Das Lösungsmittel kann dabei niedermolekularem Alkohol mit einem Alkoholgehalt von 65-75 Vol%, insbesondere von 70 Vol% aufweisen. Bei der Nasszerkleinerung und/oder Turboextraktion kommt es durch die Schneidkräfte und die Scherkräfte zu einer Zerkleinerung des Ausgangsmaterials (vorzerkleinerte Pflanzenteile), dabei werden die Zellwände vermehrt aufgerissen. Dabei werden optimaler Weise die Zellwände des Pflanzenmaterials aufgebrochen. Mit Hilfe der Scherkräfte kommt es neben einer Zerkleinerung der Pflanzenpartikel zu einer Suspendierung. In anderen Worten: Bei der Nasszerkleinerung und/oder Turboextraktion werden die Pflanzenteile unter Verwendung eines Lösungsmittels stark zerkleinert, wodurch die Oberfläche der Pflanzenteile stark vergrössert wird und so eine Zellaufschluss ermöglicht und der Kontakt zwischen dem Lösungsmittel und den Pflanzenteilen für die Extraktion optimiert wird. Neben einer zusätzlichen Zerkleinerung werden die Diffusions- und Lösungsvorgänge stark beschleunigt. Die Turboextraktion kann auch als Wirbelextraktion bezeichnet werden.

Bei der Nasszerkleinerung werden die vorzerkleinerten Pflanzenteile unter Zugabe eines niedermolekularen Alkohols in einer geeigneten Schneide- und/oder Mahlvorrichtung auf die gewünschte Grösse zerkleinert. Der niedermolekulare Alkohol kann dabei einen Alkoholgehalt von 65-95 Vol%, insbesondere von 65-75 Vol%, insbesondere von 70 Vol%, aufweisen.

Durch die Nasszerkleinerung und/oder Turboextraktion der frischen und/oder gefrorenen und/oder getrockneten Pflanzenteile der Symphytum unter Zugabe von Alkohol und/oder einem beliebigen anderen geeigneten Lösungsmittel kann die Oxidation der Pflanzenteile im Vergleich zum Mahlen und/oder Zerkleinern der Pflanzenteile an der Luft verringert werden. Dadurch wird es möglich, dass nicht nur eine oxidative Umwandlung der Wirkstoffe der Symphytum sondern auch enzymatische Reaktionen in der Extraktionsphase verringert bzw. weitgehen verhindert werden kann.

Durch das Einstellen des Alkoholgehaltes auf 65-95 Vol% wird ein Quellen, Lösen und/oder Aufschliessen der Schleimpolysaccharide unterbunden, wodurch hauptsächlich schleimfreie Wirkstoffe aus den Pflanzenteilen gelöst werden. Beispielsweise können Polyphenole, die einen Teil der Wirkstoffe der Symphytum ausmachen, ab einem Alkoholgehalt von etwa 65 Vol% besonders gut in der Extraktionsphase gelöst werden. Dadurch dass die erste, im Wesentlichen schleimfreie Extraktionsphase nur einen minimalen Gehalt an Schleimpolysacchariden aufweist, kann die Extraktion von beispielsweise Polyphenolen besonders effizient erfolgen.

Insbesondere kann der Alkoholgehalt der ersten Extraktionsphase auf 70 Vol% eingestellt werden. Bei einem solchen Alkoholgehalt kann, insbesondere in Kombination mit einer erhöhten Temperatur von 45°C - 80°C, eine antibakterielle Wirkung der ersten Extraktionsphase festgestellt werden. Auf diese Weise wird es möglich, dass die erste, im Wesentlichen schleimfreie Extraktionsphase eine keimreduzierende Wirkung entfaltet, was unter anderem für die weitere Verarbeitung und/oder für die Verwendung in einem Spezial-Extrakt vorteilhaft sein kann sowie das Einhalten gesetzlicher Vorgaben sicherstellen kann.

Bei der Herstellung der ersten Extraktionsphase kann die Temperatur der Extraktionsphase auf 45°C bis 80°C, insbesondere auf 50°C - 70°C, insbesondere auf 54-56°C erhöht werden. Durch die Erhöhung der Temperatur können zum einen die Austauschprozesse bei der Extraktion der Wirkstoffe verbessert und zum anderen können gegebenenfalls Enzyme und/oder Proteine, die sich in der Extraktionsphase befinden, (selektiv) denaturiert werden. Dadurch können enzymatische Abbau- und/oder Umwandlungsprozesse in der schleimfreien, ersten Extraktionsphase unterbunden werden.

Die schleimhaltige, zweite Extraktionsphase kann durch Mazeration, insbesondere durch Rührmazeration, der ersten Extraktionsphase hergestellt werden, wobei der Alkoholgehalt (niedermolekularer Alkohol) der zweiten Extraktionsphase auf 45 Vol% - 55 Vol%, insbesondere auf 49 Vol%-51 Vol%, insbesondere auf 50 Vol% eingestellt wird.

Das Verfahren zur Herstellung einer schleimhaltigen, zweiten Extraktionsphase als solches ist mit dem Verfahren zur Herstellung eines Spezial-Extrakts durch eine erfinderische Idee, nämlich die Verbesserung durch optimierte Verfahrensschritte bzw. Teilschritte der Herstellung eines Spezial-Extrakts aus Symphytum, verbunden. Aus diesem Grund, kann das Verfahren zur Herstellung der zweiten, schleimhaltigen Extraktionsphase aus Symphytum auch als unabhängiges Verfahren oder in Zusammenhang mit dem Verfahren zur Herstellung des Spezial-Extrakts betrachtet werden.

Die Einstellung des Alkoholgehaltes kann durch die Zugabe von Wasser zur ersten, im Wesentlichen schleimfreien, Extraktionsphase bewirkt werden. Dadurch wird die Polarität der Extraktionsphase erhöht, was zur Quellung und damit zu einem kolloidalen Lösen der Schleimpolysaccharide führt. Auf diese Weise wird es möglich, dass die zweite Extraktionsphase bzw. das Suspensionssystem aus Symphytum einen hohen Anteil an Wirkstoffen aufweist. Auf diese Weise kann eine weitere Stufe der diskontinuierlichen Extraktion erreicht werden.

Unter ständigem Rühren kann der Wassergehalt der Extraktionsphase erhöht und der Alkoholgehalt der schleimhaltigen, zweiten Extraktionsphase auf 45 Vol% - 55 Vol% eingestellt werden. Auf diese Weise wird die Polarität der zweiten Extraktionsphase im Vergleich zur ersten Extraktionsphase erhöht. Dadurch wird es möglich, dass die Schleimstoffe, wie beispielsweise die Schleimpolysaccharide, quellen und aus den Pflanzenteilen der Symphytum kolloidal gelöst und erschlossen werden. Folglich wird ein Suspensionssystem hergestellt, das der zweiten, schleimhaltigen Extraktionsphase entspricht.

Die zweite Extraktionsphase weist daher neben den kolloidal gelösten, erschlossenen Schleimpolysacchariden auch die in der ersten Extraktionsphase gut löslichen Wirkstoffe, wie beispielsweise die Polyphenole, in einer hohen Konzentration auf. Die zweite Extraktionsphase kann auch als Suspensionssystem bezeichnet werden, welches schleimig ist.

Die schleimhaltige, zweite Extraktionsphase kann bei Raumtemperatur hergestellt werden. Allerdings ist die Herstellung der zweiten Extraktphase auch bei höheren Temperaturen möglich, solang ein Zersetzung thermolabiler Schleimpolysaccharide und/oder Umwandlung der Schleimpolysaccharide nicht gefördert wird.

Das Verfahren zum Fällen, insbesondere zum differentiellen Fällen, von Schleimpolysacchariden in einem Suspensionssystem aus Symphytum als Niederschlag als solches ist mit dem Verfahren zur Herstellung eines Spezial-Extrakts durch eine erfinderische Idee, nämlich die Verbesserung bzw. Vereinfachung der Herstellung eines Spezial-Extrakts aus Symphytum, verbunden. Aus diesem Grund kann das Verfahren zum differentiellen Fällen von Schleimpolysacchariden in dem Suspensionssystem als Niederschlag auch als unabhängiges Verfahren oder im Zusammenhang mit dem Verfahren zur Herstellung des Spezial-Extrakts oder individuell im Zusammenhang mit einem der andern im diesem Text beschriebenen Verfahren betrachtet werden. Dabei können auch schleimige Suspensionssysteme aus Symphytum verwendet werden, die durch eine alternative Extraktion und nicht mit Hilfe einer diskontinuierlichen Extraktion herstellt bzw. bereitgestellt werden.

Wie bereits oben beschrieben: Das Fällen von Schleimpolysacchariden als Niederschlag kann eine differentielle Schleimfällung aufweisen. Auf diese Weise können differentiell spezifische Stoffe, wie unerwünschte und/oder wirksame Schleimstoffe, in dem Suspensionssystem gefällt und gegebenenfalls vom Extrakt abgetrennt werden.

Die differentielle Schleimfällung kann mindestens einen der folgenden Schritte aufweisen:
- erste differentielle Schleimfällung von hochmolekularen Schleimpolysacchariden als erster Niederschlag in einem bereitgestellten Suspensionssystem, Abtrennung eines ersten Niederschlags, gegebenenfalls als Teil eines Extraktionsrückstands, und Gewinnung einer ersten Extraktphase;
- zweite differentielle Schleimfällung von niedermolekularen Schleimpolysacchariden als zweiter Niederschlag in der ersten Extraktphase, sowie Abtrennung des zweiten Niederschlags und Gewinnung einer zweiten Extraktphase;
- insbesondere Trocknung des zweiten Niederschlags nach der Abtrennung.

Auf diese Weise wird es möglich, dass durch die erste differentielle Schleimfällung die hochmolekularen Schleimpolysaccharide aus dem schleimhaltigen, Suspensionssystem abgetrennt werden können. Das Abtrennen kann durch Sedimentieren und anschliessendem Abhebern des ersten Niederschlags, gegebenenfalls als Teil eines Extraktionsrückstands, und insbesondere durch Auspressen erfolgen. Die Abtrennung kann aber auch durch andere bekannte Verfahren, wie beispielsweise Filtrieren, Dekantieren, Sieben, Zentrifugieren und/oder Pressen erfolgen.

Das Raffinat, welches neben dem ersten Niederschlag auch den Extraktionsrückstand aufweisen kann, kann für das Verfahren zur Herstellung des Spezial-Extrakts recycliert werden. Dadurch wird ermöglicht, dass die hochmolekularen Schleimpolysaccharide des ersten Niederschlags gegebenenfalls einer weiteren Verwendung zugeführt werden können.

Mit Hilfe der ersten differentiellen Schleimfällung kann die erste Extraktphase spezifisch von den hochmolekularen Schleimpolysacchariden abgetrennt werden. Die hochmolekularen Schleimpolysaccharide penetrieren im Gegensatz zu niedermolekularen Schleimpolysacchariden nicht durch die Haut und können daher derzeit nicht als potenzielle Wirkstoffkomponente verwendet werden. Die hochmolekularen Schleimpolysaccharide besitzen aus heutiger Sicht kein therapeutisches Potenzial. Dadurch werden zum einen die unwirksamen hochmolekularen Schleimpolysaccharide aus dem Extrakt entfernt und zum anderen wird die erste Extraktphase dünnflüssiger als das Suspensionssystem und damit besser verarbeitbar.

Bei der zweiten differentiellen Schleimfällung können spezifisch die niedermolekularen Schleimpolysaccharide, welche als Wirkstoffe für das Spezial-Extrakt von Interesse sind, nach dem Abtrennen der hochmolekularen Schleimpolysaccharide aus der dünnflüssigen ersten Extraktphase gefällt und als disperser, zweiter Niederschlag bzw. Präzipitat von der fluiden, zweiten Extraktphase getrennt werden. Der zweite Niederschlag kann durch ein bekanntes Verfahren, wie beispielsweise Filtrieren, Dekantieren, Sieben, Zentrifugieren und/oder Pressen von der zweiten Extraktphase getrennt und so gewonnen werden. Dadurch entsteht ein zweiter, kolloid-disperser Niederschlag, welcher die niedermolekularen Schleimpolysaccharide aufweist und der meist in kolloidal gelöster Form in der zweiten Extraktphase vorliegt. In getrockneter Form kann der zweite Niederschlag als sehr feinpulvriger Niederschlag vorliegen.

Der zweite Niederschlag kann nach dem Abtrennen getrocknet werden. Dadurch wird es möglich, dass der Niederschlag im getrockneten Zustand gelagert werden kann. Der zweite Niederschlag kann beispielsweise lyophilisiert werden. Da Lyophilisierung eine besonders schonende Trocknungsmethode ist, die bei geringem Druck durchgeführt wird, kann eine Oxidation des zweiten Niederschlags unterbunden werden. Es ist jedoch auch jede andere Trocknungsmethode zur Trocknung des zweiten Niederschlags denkbar.

Bei der ersten differentiellen Schleimfällung kann der pH-Wert des schleimigen, Suspensionssystems aus Symphytum auf 4 bis 5 eingestellt werden, wobei das Suspensionssystem vor der Fällung einen pH-Wert von 6 bis 6,5 aufweisen kann. Die ausgefallenen hochmolekularen Schleimpolysaccharide können wie bereits beschrieben abgetrennt werden. Nach dem Abtrennen des ersten Niederschlags von der ersten Extraktphase weist die erste Extraktphase eine geringere Viskosität auf als das schleimige Suspensionssystem. Dadurch kann die erste Extraktphase gut weiterverarbeitet werden und die Handhabbarkeit für weitere Auswasch- und/oder Fällungsprozesse wird verbessert.

Es sei angemerkt, dass während der ersten differentiellen Schleimfällung durch das Einstellen des pH-Wert auf 4 - 5 die Polarität der Extraktionsphase verändert wird. Dadurch kann eine zusätzliche Extraktion von Wirkstoffen aus den im Suspensionssystem vorhanden suspendieren Pflanzenteilen und somit einer Erhöhung der Wirkstoffkonzentration in der erste Extraktphase ermöglicht werden. Mit anderen Worten: Durch die zusätzliche Extraktion, die mit der ersten differenziellen Schleimfällung und die damit verbundene Veränderung der Polarität einhergehen kann, kann die mehrstufige, diskontinuierliche Extraktion durch einen weiteren Verfahrensschritt bzw. eine weitere pH-modifizierte Stufe optimiert werden. Im Fall, dass das Suspensionssystem aus Symphytum durch die beschriebene diskontinuierliche Extraktion bereitgestellt wird, wird in einem ersten Schritt die erste Extraktionsphase hergestellt. In dieser ersten Extraktionsphase mit einem relativ hohen Alkoholgehalt werden, wie beschrieben, spezifisch alkohollösliche Extraktivstoffe aus den Pflanzenteilen gelöst. In einem zweiten Schritt wird die zweite Extraktionsphase mit einem niedrigeren Alkoholgehalt hergestellt, dabei werden die Schleimpolysaccharide durch Quellung aufgeschlossen und kolloidal gelöst. In einem dritten Schritt können aufgrund der erneuten Änderung der Polarität weiter Extraktivstoffe in einem sauren Milieu aus den Pflanzenteilen gelöst werden. Der dritte Schritt ist natürlich nur möglich, wenn der Extraktionsrückstand nicht vor der ersten differenziellen Schleimfällung entfernt wurde.

Bei der zweiten differentiellen Schleimfällung von niedermolekularen Schleimpolysacchariden in der ersten Extraktphase kann der pH-Wert der ersten Extraktphase auf 2.5 - 3.5 eingestellt werden. Auf diese Weise bildet sich ein feiner, kolloid-disperser, zweiter Niederschlag, der zunächst in Schwebe bleiben kann, wodurch eine gute Abtrennung ohne Klumpenbildung ermöglicht wird. Der feine zweite Niederschlag kann eine hellbeige Farbe aufweisen. Aufgrund der feinen Oberflächenstruktur des gefällten zweiten Niederschlags kann der Niederschlag effizient von unerwünschten Begleitstoffen, wie beispielsweise Pyrrolizidin-Alkaloiden, gereinigt werden. Des Weiteren kann der feine, zweite Niederschlag durch eines der bereits beschriebenen und bekannten Verfahren von der ersten Extraktphase abgetrennt werden.

Im Gegensatz dazu bildet sich durch eine Fällung der Schleimpolysaccharide in einem einzigen Schritt, beispielsweise mit Ethanol, ein gummiartiges dunkelbraunes Agglomerat. Das Agglomerat, in dieser Form, verunmöglicht ein allfälliges Entfernen von Pyrrolizidin-Alkaloiden.

Der pH-Wert des schleimigen Suspensionssystems, insbesondere der zweiten, schleimhaltige Extraktionsphase und/oder der erste Extraktphase kann mit Hilfe eines Katalysators und/oder mit Hilfe einer Säure eingestellt werden. Der Katalysator, auch Kationenaustauscher genannt, kann ein saurer Katalysator auf gelförmiger Polymerbasis sein. Auf diese Weise kann der Katalysator einfach, beispielsweise durch Absieben, aus der Extraktionslösung entfernt werden.

Der saure Katalysator kann Sulfonsäuregruppen auf der Polymeroberfläche aufweisen. Auf diese Weise kann ein Ionenaustausch auf der Oberfläche des Katalysators stattfinden, wobei Kationen gebunden werden. Durch die Reduktion des pH-Werts fallen selektiv Schleimpolysaccharide aus dem Extrakt (Suspensionssystem und/oder ersten Extraktphase) aus und bilden einen (ersten und/oder zweiten) Niederschlag. Zusätzlich können auch ionische Pyrrolizidin-Alkaloide durch Ionenaustausch am Katalysator angelagert werden, wodurch es zu einer Teilreduktion von unerwünschten Pyrrolizidin-Alkaloiden im Extrakt, insbesondere in der ersten Extraktphase und/oder in der zweiten Extraktionsphase, kommt. Mit anderen Worten: der pH-Wert des Suspensionssystems und/oder der ersten Extraktphase kann mit Hilfe eines Kationenaustauschers in saurer Form eingestellt werden, wobei der Kationenaustauscher auf der Basis eines organischen Polymerharzes ausgestaltet sein kann und wobei der Kationenaustauscher insbesondere Sulfonsäuregruppen auf der Polymeroberfläche aufweisen kann.

In weiteren Ausführungsformen kann die Säure zum Einstellen des pH-Wertes eine Mineralsäure oder organische Säure sein. Auch hier kommt es durch die Senkung des pH-Wertes zu einer selektiven Fällung der Schleimpolysaccharide wie bereits beschrieben, jedoch ohne Abreicherung unerwünschter Pyrrolizidin-Alkaloide.

Bei einer Verwendung des sauren Katalysators für das Einstellen des pH-Wertes des Suspensionssystem auf 4-5 für die erste, differentielle Schleimfällung kann die Extraktionslösung bereits von Pyrrolizidin-Alkaloiden vorgereinigt werden, da der saure Katalysator einen Teil der gelösten Pyrrolizidin-Alkaloide auf der Katalysatoroberfläche binden kann.

Die Reinigung des zweiten Niederschlags durch die selektive Entfernung von Pyrrolizidin-Alkaloiden aus dem zweiten Niederschlag kann die folgenden Schritte aufweisen:
- Resuspendierung des zweiten Niederschlags in einer Waschlösung, insbesondere in einer Waschlösung aufweisend einen sauren, niedermolekularen Alkohol,
- Abtrennen des gewaschenen zweiten Niederschlags von der Waschlösung.

Beim Resuspendieren des zweiten Niederschlags in der Waschlösung bleiben die Schleimpolysaccharide des Niederschlags unlöslich und die Pyrrolizidin-Alkaloide werden in der Waschlösung gelöst und können so aus dem Niederschlag gewaschen bzw. extrahiert werden. Dies kann dadurch ermöglicht werden, dass der Alkoholgehalt der Waschlösung 65-95 Vol%, insbesondere 78 Vol% - 82 Vol%, beträgt. Dadurch kann der von Pyrrolizidin-Alkaloiden gereinigte zweite Niederschlag nach dem Waschen von der Waschlösung auf eine bekannte Art und Weise, wie beispielsweise Sedimentieren und Abhebern oder ähnliche Verfahrensschritte, abgetrennt werden.

Der pH-Wert der sauren Waschlösung kann 2 - 4, insbesondere 2.5 - 3.5, insbesondere mindestens 3, betragen.

Beim Resuspendieren kann ein Verhältnis von Niederschlag zu Waschlösung von 1:10 verwendet werden. Das Verhältnis bezieht sich auf die Masse des Niederschlags und der Waschlösung.

Die Reinigung des zweiten Niederschlags durch die selektive Entfernung von Pyrrolizidin-Alkaloiden aus dem zweiten Niederschlag kann alternativ auch die folgenden Schritte aufweisen:
- Resuspendierung des zweiten Niederschlags, insbesondere eines lyophilisierten zweiten Niederschlags, in hochprozentigem, niedermolekularem Alkohol, insbesondere Ethanol,
- Versetzen des resuspendierten zweiten Niederschlags mit einem Katalysator insbesondere einem sauren Katalysator auf gelförmiger Polymerbasis, wobei der Katalysator insbesondere Sulfonsäuregruppen auf der Polymeroberfläche aufweist, und
- Abtrennen des Katalysators vom resuspendierten zweiten Niederschlag und anschliessender Rückgewinnung des gereinigten zweiten Niederschlags aus der gereinigten Suspension.

Dadurch wird es möglich, dass der zweite Niederschlag von Pyrrolizidin-Alkaloiden gereinigt werden kann. Die Schleimpolysaccharide lösen sich in hochprozentigem Alkohol nicht, jedoch sind die Pyrrolizidin-Alkaloide gut in Alkohol löslich. Auf diese Weise können die Pyrrolizidin-Alkaloide aus dem zweiten Niederschlag herausgelöst werden, ohne dass sich die wirksamen Schleimpolysaccharide aus dem zweiten Niederschlag lösen. Durch das Versetzen des resuspendierten zweiten Niederschlags mit dem sauren Katalysator können die Pyrrolizidin-Alkaloide wie bereits beschrieben, mittels Ionenaustausch aus der Suspension am Katalysator gebunden und entfernt werden. Die Menge des saueren Katalysators kann an die Menge des resuspendierten zweiten Niederschlags angepasst werden, wobei insbesondere gleiche Mengen Katalysator und Niederschlag für die Reinigung verwendet werden können. Gleiche Menge bedeutet in diesem Zusammenhang gleiche Masse. Der Katalysator kann vom feinen resuspendierten zweiten Niederschlag abgetrennt werden und der zweite Niederschlag kann anschliessend von der flüssigen hochprozentigen Alkohollösung abgetrennt werden. Die Abtrennung kann wie bereits beschrieben durch ein bekanntes Verfahren, wie beispielsweise Sedimentierung und Abhebern oder Dekantieren oder Ähnliches erfolgen.

Der hochprozentige, niedermolekulare Alkohol kann 80 Vol% bis 98 Vol%, insbesondere 96 Vol% niedermolekularen Alkohol aufweisen.

Beim Resuspendieren des zweiten Niederschlags kann die Menge (Masse) des Niederschlags an die Menge des Alkohols angepasst werden. Dabei kann beispielsweise ein Teil des zweiten Niederschlags in 5 bis 10 Teilen Alkohol resuspendiert werden.

Das Resuspendieren und Pyrrolizidin-Alkaloid-Reinigen kann gegebenenfalls mit dem gereinigten zweiten Niederschlag wiederholt werden, wodurch der Pyrrolizidin-Alkaloid-Gehalt des Niederschlags weiter gesenkt werden kann.

Der gereinigte zweite Niederschlag kann maximal 1 ppm, insbesondere maximal 0.5 ppm, insbesondere maximal 0.1 ppm Pyrrolizidin-Alkaloide aufweisen. Dadurch wird es möglich, dass der gereinigte zweite Niederschlag aus toxikologischer Sicht ohne Bedenken im Spezial-Extrakt eingesetzt werden kann.

Die selektive Entfernung von Pyrrolizidin-Alkaloiden (PA) aus der zweiten Extraktphase kann mindestens einen der folgenden Schritte aufweisen:
- Pyrrolizidin-Alkaloid-Reinigung der zweiten Extraktphase mit Hilfe einer stationären Phase, wobei die stationäre Phase insbesondere einen Kationenaustauscher in saurer Form aufweist;
- Einstellen des pH-Wertes der Pyrrolizidin-Alkaloid-gereinigten zweite Extraktphase, auf 5.5-7, insbesondere auf 6.2-6.5; und
- Aufkonzentrierung der Pyrrolizidin-Alkaloid-gereinigten zweite Extraktphase, insbesondere mittels Destillation
wobei die gereinigte, insbesondere aufkonzentrierte, zweite Extraktphase, insbesondere die gereinigte zweite Extraktphase, maximal 0.5 ppm Pyrrolizidin-Alkaloide, insbesondere maximal 0.25 ppm Pyrrolizidin-Alkaloide, insbesondere maximal 0.1 ppm Pyrrolizidin-Alkaloide, aufweist.

Die Pyrrolizidin-Alkaloid-Reinigung der zweiten Extraktphase mit Hilfe einer stationären Phase kann beispielsweise Durch die chromatographische, insbesondere säulenchromatographische, Reinigung realisiert werden. Durch die chromatographische, insbesondere säulenchromatographische, Reinigung der Extraktphase, insbesondere der zweiten Extraktphase, können Pyrrolizidin-Alkaloide aus der zweiten Extraktphase entfernt werden. Die Entfernung der Pyrrolizidin-Alkaloide kann insbesondere mittels des bereits beschrieben sauren Katalysators auf gelförmiger Polymerbasis erfolgen. Dabei kann der Katalysator Sulfonsäuregruppen auf der Polymeroberfläche aufweisen. Auf diese Weise kann ein Ionenaustausch stattfinden, der sich durch die folgende Formel beschreiben lässt:

Katalysator-H + PA⁺ → Katalysator-PA + H⁺

wobei Katalysator-H der saure Katalysator, PA⁺ ein ionisches Pyrrolizidin-Alkaloide, Katalysator-PA eine gebundene Form von Katalysator und Pyrrolizidin-Alkaloide und H⁺ ein Proton ist. Dadurch wird es möglich, dass die Pyrrolizidin-Alkaloide am Katalysator gebunden werden und die zweite Extraktphase von den Pyrrolizidin-Alkaloiden gereinigt wird. Da die zweite Extraktphase nach dem Abtrennen des Niederschlags eine relativ geringe Viskosität aufweist, eignet sich die zweite Extraktphase ohne Fällungsreaktion gut für die chromatographische Reinigung mit einem Katalysator.

Nach dem Reinigen der zweiten Extraktphase kann der pH-Wert der zweiten Extraktphase auf 5.5-7, insbesondere auf 6.2-6.5 eingestellt werden. Dadurch kann die Weiterverarbeitung, wie beispielsweise das Auskonzentrieren, der gereinigten Extraktphase verbessert werden.

Des Weiteren kann die gereinigte zweite Extraktphase aufkonzentriert werden. Durch die damit einhergehende Verringerung des Volumens bei gleichbleibender Stoffmenge an Wirkstoffen bzw. Extraktivstoffen, kann die Wirkstoffkonzentration der Pyrrolizidin-Alkaloid-gereinigten zweiten Extraktphase erhöht werden. Auf diese Weise kann die Herstellung des Spezial-Extrakts verbessert werden und ein Droge-Extrakt-Verhältnis (DEV) definiert werden. Das Aufkonzentrieren kann beispielsweise mit Hilfe einer Destillation, insbesondere einer Vakuumdestillation, durchgeführt werden.

Beispielsweise kann durch die vorgängige Aufkonzentrierung der gereinigten Extraktphase, insbesondere der gereinigten zweiten Extraktphase, der Alkoholgehalt des Spezial-Extrakts nach dem Mischen reduziert werden. Dadurch kann die Stabilität des Spezial-Extrakts bei der weiteren Verarbeitung in eine galenische Zubereitung verbessert werden.

In einer Ausführungsform kann das Verfahren zur Herstellung eines Spezial-Extrakts aus Symphytum mit einem Pyrrolizidin-Alkaloid-Gehalt von maximal 0.5 ppm, insbesondere maximal 0.1 ppm, die folgenden Schritte aufweisen:
- Bereitstellung einer suspendierten, ersten, schleimfreien Extraktionsphase aus Symphytum;
- Herstellung einer zweiten, schleimhaltigen Extraktionsphase;
- erste differentielle Schleimfällung von hochmolekularen Schleimpolysacchariden als erster Niederschlag in einer zweiten, schleimhaltigen Extraktionsphase, Abtrennung des ersten Niederschlags und eines Extraktionsrückstands als Raffinat und Gewinnung einer ersten Extraktphase;
- zweite differentielle Schleimfällung von niedermolekularen Schleimpolysacchariden als zweiter Niederschlag aus der ersten Extraktphase, Abtrennung des zweiten Niederschlags und Gewinnung einer zweiten Extraktphase;
- selektive Entfernung von Pyrrolizidin-Alkaloiden aus dem zweiten Niederschlag;
- selektive Entfernung von Pyrrolizidin-Alkaloiden aus der zweiten Extraktphase; und
- Vereinigen des Pyrrolizidin-Alkaloid-gereinigten zweiten Niederschlags, und der Pyrrolizidin-Alkaloid-gereinigten zweiten Extraktphase, insbesondere einer aufkonzentierten Pyrrolizidin-Alkaloid-gereinigten zweiten Extraktphase, zum Spezial-Extrakt.

Auf diese Weise wird ein Verfahren zur Verfügung gestellt, mit dem ein Spezial-Extrakt mit einem Pyrrolizidin-Alkaloid-Gehalt von maximal 0.5 ppm, insbesondere maximal 0.25 ppm, hergestellt werden kann.

Mindestens einer der folgenden Schritte und/oder Teilschritte:
- Bereitstellen eines Suspensionssystems aus Symphytum;
- erste differentielle Schleimfällung von hochmolekularen Schleimpolysacchariden;
- zweite differentielle Schleimfällung von niedermolekularen Schleimpolysacchariden;
- selektive Entfernung von Pyrrolizidin-Alkaloiden aus dem zweiten Niederschlag und/oder der zweiten Extraktphase
kann unter Schutzgas durchgeführt werden. Dadurch wird es möglich, dass eine Oxidation der Ausgangsstoffe oder Zwischenprodukte unterbunden bzw. verringert wird. Des Weiteren können ungewollte enzymatische Reaktionen minimiert werden.

In einem weiteren Aspekt betrifft die Erfindung ein Spezial-Extrakt, welches mit dem beschrieben Verfahren herstellbar ist.

Das Spezial-Extrakt aus Symphytum kann in der Therapie angewendet werden.

Das Spezial-Extrakt aus Symphytum kann insbesondere zur äusserlichen Anwendung in der Therapie verwendet werden.

Es ist auch möglich, dass das Spezial-Extrakt aus Symphytum für kosmetische Zwecke angewendet werden kann.

Das Spezial-Extrakt kann insbesondere in der Therapie von Schmerzen, Entzündungen, Schwellungen, Bewegungseinschränkungen, Prellungen, Zenungen, Vertauchungen, Gelenkarthrose, Rückenschmerzen, Muskelbeschwerden und/oder Gelenkbeschwerden angewendet werden.

Wie bereits beschrieben betrifft ein Aspekt der Erfindung zur Verbesserung der Herstellung eines Spezial-Extrakts aus Symphytum ein Verfahren zur Herstellung einer im Wesentlichen schleimfreien, suspendierten, ersten Extraktionsphase aus Symphytum aufweisend die folgenden Schritte:
- Bereitstellen von frischen, gefrorenen und/oder getrockneten Pflanzenteilen der Symphytum;
- Versetzen der Pflanzenteile mit einem niedermolekularem Alkohol, insbesondere mit Ethanol;
- Einstellen des Alkoholgehaltes der ersten Extraktionsphase auf 65-75 Vol%, insbesondere auf 70 Vol%.

Ein weiterer Aspekt der Erfindung zur Verbesserung der Herstellung eines Spezial-Extrakts aus Symphytum betrifft ein Verfahren zur Herstellung einer zweiten, schleimhaltigen Extraktionsphase aus einer bereitgestellten ersten, im Wesentlichen schleimfreien Extraktionsphase aus Symphytum, wobei die zweite, schleimhaltige Extraktionsphase durch Mazeration, insbesondere durch Rührmazeration, der ersten Extraktionsphase hergestellt wird, wobei der Alkoholgehalt der Extraktionsphase auf 45 Vol% - 55 Vol%, insbesondere auf 50 Vol% eingestellt wird, wie bereits beschrieben.

Ein weiterer Aspekt der Erfindung zur Verbesserung der Herstellung eines Spezial-Extrakts aus Symphytum betrifft ein Verfahren zum Fällen von Schleimpolysacchariden (SPS) aufweisend eine differentiellen Schleimfällung, wobei die differentielle Schleimfällung folgende Schritte aufweist:
- erste differentielle Schleimfällung von hochmolekularen Schleimpolysacchariden als erster Niederschlag in einem Suspensionssystem aus Symphytum, Abtrennung des ersten Niederschlags und gegebenenfalls eines Extraktionsrückstands als Raffinat und Gewinnung einer ersten Extraktphase;
- zweite differentielle Schleimfällung von niedermolekularen Schleimpolysacchariden als zweiter Niederschlag aus der ersten Extraktphase, Abtrennung des zweiten Niederschlags und Gewinnung einer zweiten Extraktphase.

Ein weiterer Aspekt der Erfindung zur Verbesserung der Herstellung eines Spezial-Extrakts aus Symphytum betrifft ein Verfahren zur selektive Entfernung von Pyrrolizidin-Alkaloiden (PA) von einem aus einem Suspensionssystem aus Symphytum gefällten und von einem Extrakt getrennten Niederschlag aufweisend die folgenden Schritte:
- Resuspendierung des gefällten und abgetrennten Niederschlags in einer Waschlösung, insbesondere in einer Waschlösung aufweisend einen sauren, niedermolekularen Alkohol, wobei der Alkohol insbesondere einen pH-Wert von 2-4 aufweist, und
   Abtrennen des gewaschenen Niederschlags von der Waschlösung;
   oder
- Resuspendierung des gefällten und abgetrennten Niederschlags in hochprozentigem, niedermolekularem Alkohol, insbesondere Ethanol, Versetzen des resuspendierten Niederschlags mit einem Katalysator insbesondere einem sauren Katalysator auf gelförmiger Polymerbasis, wobei der Katalysator insbesondere Sulfonsäuregruppen auf der Polymeroberfläche aufweist, und Abtrennen des Katalysators vom resuspendierten Niederschlag und anschliessender Rückgewinnung des gereinigten Niederschlags aus der Suspension;
wobei das Resuspendieren und Reinigen mit dem gereinigten Niederschlag wiederholbar ist; und
wobei der gereinigte Niederschlag insbesondere maximal 1 ppm, insbesondere maximal 0.5 ppm, insbesondere maximal 0.1 ppm, Pyrrolizidin-Alkaloide aufweist.

In einem weiteren Aspekt betrifft die Erfindung zur Verbesserung der Herstellung eines Spezial-Extrakts aus Symphytum ein wie bereits beschriebenem Verfahren zur selektive Entfernung von Pyrrolizidin-Alkaloiden aus einer von einem Niederschlag in einem Suspensionssystem aus Symphytum gefällten und abgetrennten Extraktphase, aufweisend mindestens einen der folgenden Schritte:
- Reinigung der Extraktphase mit Hilfe einer stationären Phase, wobei die stationäre Phase insbesondere einen Kationenaustauscher in saurer Form aufweist;
- Einstellen des pH-Wertes der gereinigten Extraktphase, auf 5.5-7, insbesondere auf 6.2-6.5; und
- Aufkonzentrierung der gereinigten Extraktphase, insbesondere mittels Destillation, insbesondere mittels Vakuumdestillation,
wobei die gereinigte, insbesondere aufkonzentrierte, Extraktphase, maximal 0.5 ppm Pyrrolizidin-Alkaloide, insbesondere maximal 0.1 ppm Pyrrolizidin-Alkaloide aufweist.

Weitere bevorzugte Ausführungsformen gehen aus den abhängigen Patentansprüchen hervor. Dabei sind Merkmale der Verfahrensansprüche sinngemäss mit den Verwendungsansprüchen kombinierbar und umgekehrt. Auch sind die unabhängigen Verfahren miteinander in beliebiger Kombination kombinierbar.

### Beispiel

Unterirdische Teile von Symphytum officinale L. in einer Schnittgrösse von 5 mm - 10 mm werden in Ethanol 70% Vol. unter Rühren mit einem Turbomischer bei kontinuierlich ansteigender Temperatur auf 55°C einer Nasszerkleinerung zu Partikelgrössen von 0,1 mm - 1 mm unterzogen. Auf diese Weise wird eine erste, im Wesentlichen schleimfreie Extraktionsphase 1 hergestellt. Im Anschluss daran erfolgt bei Raumtemperatur durch Zugabe von gereinigtem Wasser während 180 Minuten eine Bewegungsmazeration, wodurch die zweite, schleimhaltige Extraktionsphase 2 hergestellt wird, die eine höhere Polarität aufweist als die erste, schleimfreie Extraktionsphase 1.

Unter weiterem Rühren wird ein erster, differentieller Schleimfällungsschritt durch Zugabe von saurem Katalysator in protonierte (H⁺) Form eingeleitet. Der Katalysator ist ein saurer Katalysator auf gelförmiger Polymerbasis und weist auf der Polymeroberfläche Sulfonsäuregruppen auf. Dabei wird der pH-Wert der zweiten Extraktionsphase 2 auf 4-5 eingestellt und hochmolekulare Schleimpolysaccharide werden als erster Niederschlag N1 gefällt. Nach einem weiteren Extraktionsschritt (pH-modifiziert) erfolgt ein Trennen der flüssigen Extraktphase, auch erstes Extraktphase A genannt, von dem dispersen Extraktionsrückstand durch Auspressen der Sedimentphase bzw. des Extraktionsrückstands. Durch diese Verfahrensweise entsteht die erste Extraktphase A, welche als Filtrat einem weiteren Protonenaustausch unter Zusatz des oben genannten sauren Katalysators während mindestens 60 Minuten, bis zum stabilen Erreichen von pH 2,5-3,5, unterzogen wird. Einhergehend mit der Senkung des pH-Wertes und unter Rührung der ersten Extraktphase A kommt es zur quantitativen Fällung der Schleimpolysaccharide als zweiter Niederschlag N2. Der saure Katalysator wird anschliessend abgesiebt und der disperse, zweite Niederschlag N2, auch Schleimpräzipitat genannt, durch Zentrifugieren des dispersen Systems von der entstandenen zweiten Extraktphase B entfernt.

Der sich nach bisheriger Vorgehensweise ergebenden zweite Niederschlag N2, wird in salzsaurem Ethanol (Ethanol 80% Vol. mit Salzsäure auf pH 3.0 eingestellt) resuspendiert und anschliessend zentrifugiert. Dieser Auswaschprozess wird am Zentrifugat durch dreimaliges Wiederholen fortgesetzt, wobei im dabei entstandenen Pyrrolizidin-Alkaloid-gereinigten, zweiten Niederschlag N2 Pyrrorizidinalkaloid-Restwerte von maximal 1 ppm festgestellt wurden (LC-MS/MS).

Die filtrierte zweite Extraktphase B wird nun als mobile Phase in einer Chromatographiesäule, welche als stationäre Phase mit saurem Kationenaustauscher befüllt wird, chromatographiert. Das Eluat enthält Rest-Pyrrolizidin-Alkaloide von maximal 0.1 ppm (LC-MS/MS). Als stationäre Phase dient der oben beschriebene saure Katalysator auf Polymerbasis. Das Eluat wird mit Natronlauge auf pH 6.2 eingestellt, durch Vakuumdestillation konzentriert und mit Ethanol (96% Vol). versetzt. In der somit stabilisierten und gereinigten zweiten Extraktphase B wird der Pyrrolizidin-Alkaloide-gereinigte, zweite Niederschlag N2 suspendiert und gelöst und anschliessend zudem mit gereinigtem Wasser ergänzt.

Die vorgenannte Verfahrensweise ergibt einen Pyrrolizidin-Alkaloide-gereinigten Spezial-Extrakt SE, auch SpezialFluidextrakt genannt, aus Symphytum, mit einem Drogen/Extrakt-Verhältnis (DEV) von 1 : 4.

## Patentansprüche

1. Verfahren zur Herstellung eines Spezial-Extrakts (SE) aus Symphytum, wobei das Verfahren die folgenden Schritte aufweist:
- Bereitstellen eines Suspensionssystems (SS) aus Symphytum;
- differentielles Fällen von Schleimpolysacchariden (SPS), wobei die differentielle Fällung die folgenden Schritte aufweist:
- erste differentielle Schleimfällung von hochmolekularen Schleimpolysacchariden (SPS) als erster Niederschlag (N1) in dem Suspensionssystem (SS), Abtrennung des ersten Niederschlags (N1) und eines Extraktionsrückstands als Raffinat (R) und Gewinnung einer ersten Extraktphase (A);
- zweite differentielle Schleimfällung von niedermolekularen Schleimpolysacchariden (SPS) als zweiter Niederschlag (N2) aus der ersten Extraktphase (A), Abtrennung des zweiten Niederschlags (N2) und Gewinnung einer zweiten Extraktphase (B);
- selektive Entfernung von Pyrrolizidin-Alkaloiden (PA) aus dem zweiten Niederschlag (N2) und/oder der zweiten Extraktphase (B); und
- Vereinigen des zweiten Niederschlags (N2), insbesondere des Pyrrolizidin-Alkaloide-gereinigten zweiten Niederschlags (N2), und der zweiten Extraktphase (B), insbesondere der Pyrrolizidin-Alkaloide-gereinigten zweiten Extraktphase (B), zum Spezial-Extrakt (SE).

2. Verfahren gemäss Anspruch 1, wobei für die erste differentielle Schleimfällung der pH-Wert des Suspensionssystems auf 4-5 eingestellt wird.

3. Verfahren gemäss einem der Ansprüche 1-2, wobei für die zweite differentielle Schleimfällung von niedermolekularen Schleimpolysacchariden (SPS) der pH-Wert der ersten Extraktphase (A) auf 2.5 - 3.5 eingestellt wird.

4. Verfahren gemäss einem der Ansprüche 2-3, wobei der pH-Wert des Suspensionssystems und/oder der ersten Extraktphase (A) mit Hilfe eines Kationenaustauschers in saurer Form eingestellt wird, wobei der Kationenaustauscher auf der Basis eines organischen Polymerharzes ausgestaltet ist und wobei der Kationenaustauscher insbesondere Sulfonsäuregruppen auf der Polymeroberfläche aufweist.

5. Verfahren gemäss einem der Ansprüche 1-4, wobei das Spezial-Extrakts (SE) einen Pyrrolizidin-Alkaloid-Gehalt von maximal 0.5 ppm, insbesondere maximal 0.1 ppm aufweist.

6. Verfahren gemäss einem der Ansprüche 1 - 5, wobei das Suspensionssystems (SS) aus Symphytum mittels diskontinuierlicher Extraktion, aufweisend die folgenden Schritte:
- Bereitstellung einer suspendierten, ersten, schleimfreien Extraktionsphase (1) aus Symphytum; und
- Herstellung einer zweiten, schleimhaltigen Extraktionsphase (2),
herstellbar ist.

7. Verfahren gemäss Anspruch 6, wobei die suspendierte, erste Extraktionsphase (1) durch die folgenden Schritte bereitstellbar ist:
- Bereitstellen von Pflanzenteilen der Symphytum;
- Versetzen der Pflanzenteile mit einem niedermolekularem Alkohol, insbesondere mit Ethanol;
- Einstellen des Alkoholgehaltes der ersten Extraktionsphase (1) auf 65-75 Vol%, insbesondere auf 70 Vol%.

8. Verfahren gemäss einem der Ansprüche 7, wobei die bereitgestellten und mit dem niedermolekularem Alkohol versetzen Pflanzenteile von Symphytum mittels Nasszerkleinerung und/oder Turboextraktion suspendiert werden.

9. Verfahren gemäss einem der Ansprüche 6 - 8, wobei die zweite Extraktionsphase (2) durch Mazeration, insbesondere durch Rührmazeration, der ersten Extraktionsphase (1) hergestellt wird, wobei der Alkoholgehalt der zweiten Extraktionsphase (2) auf 45 Vol% - 55 Vol%, insbesondere auf 50 Vol% eingestellt wird.

10. Verfahren gemäss einem der Ansprüche 1 - 9, wobei selektive Entfernung von Pyrrolizidin-Alkaloiden (PA) aus dem zweiten Niederschlag (N2) folgende Schritte aufweist:
- Resuspendierung des zweiten Niederschlags (N2) in einer Waschlösung, insbesondere in einer Waschlösung aufweisend einen sauren, niedermolekularen Alkohol, wobei der Alkohol insbesondere einen pH-Wert von 2-4 aufweist, und
Abtrennen des gewaschenen zweiten Niederschlags (N2) von der Waschlösung;
oder
- Resuspendierung des zweiten Niederschlags (N2) in hochprozentigem, niedermolekularem Alkohol, insbesondere Ethanol,
Versetzen des resuspendierten zweiten Niederschlags (N2) mit einem Katalysator insbesondere einem sauren Katalysator auf gelförmiger Polymerbasis, wobei der Katalysator insbesondere Sulfonsäuregruppen auf der Polymeroberfläche aufweist, und
Abtrennen des Katalysators vom resuspendierten zweiten Niederschlag (N2) und anschliessender Rückgewinnung des gereinigten zweiten Niederschlags (N2) aus der gereinigten Suspension;
wobei das Resuspendieren und Pyrrolizidin-Alkaloid-Reinigen mit dem gereinigten zweiten Niederschlag (N2) wiederholbar ist; und
wobei der gereinigte zweite Niederschlag (N2) insbesondere maximal 1 ppm Pyrrolizidin-Alkaloide (PA) aufweist.

11. Verfahren gemäss einem der Ansprüche 1 - 10, wobei die selektive Entfernung von Pyrrolizidin-Alkaloiden (PA) aus der zweiten Extraktphase (B) mindestens einen der folgenden Schritte aufweist:
- Pyrrolizidin-Alkaloid-Reinigung der zweiten Extraktphase (B) mit Hilfe einer stationären Phase, wobei die stationäre Phase insbesondere einen Kationenaustauscher in saurer Form aufweist;
- Einstellen des pH-Wertes der Pyrrolizidin-Alkaloid-gereinigten zweite Extraktphase (B), auf 5.5-7, insbesondere auf 6.2-6.5; und
- Aufkonzentrierung der Pyrrolizidin-Alkaloid-gereinigten zweite Extraktphase (B), insbesondere mittels Destillation
wobei die Pyrrolizidin-Alkaloid-gereinigte und eventuell aufkonzentrierten zweite Extraktphase (B), maximal 0.5 ppm Pyrrolizidin-Alkaloide (PA), insbesondere maximal 0.25 ppm Pyrrolizidin-Alkaloide (PA), insbesondere maximal 0.1 ppm Pyrrolizidin-Alkaloide (PA), aufweist.

12. Verfahren gemäss einem der Ansprüche 1 - 11, wobei mindestens einer der Schritte:
- Bereitstellen eines Suspensionssystems (SS) aus Symphytum;
- erste differentielle Schleimfällung von hochmolekularen Schleimpolysacchariden (SPS);
- zweite differentielle Schleimfällung von niedermolekularen Schleimpolysacchariden (SPS);
- selektive Entfernung von Pyrrolizidin-Alkaloiden (PA) aus dem zweiten Niederschlag (N2) und/oder der zweiten Extraktphase (B)
unter Schutzgas durchgeführt wird.

13. Spezial-Extrakt herstellbar gemäss einem der Ansprüche 1-12.

14. Spezial-Extrakt herstellbar gemäss einem der Ansprüche 1-12 zur Anwendung in der Therapie.

15. Spezial-Extrakt herstellbar gemäss einem der Ansprüche 1-12 zur Anwendung in der Therapie von Schmerzen, Entzündungen, Schwellungen, Bewegungseinschränkungen, Prellungen, Zerrungen, Vertauchungen, Gelenkarthrose, Rückenschmerzen, Muskelbeschwerden und/oder Gelenkbeschwerden.

16. Verfahren zur Herstellung einer suspendierten, ersten Extraktionsphase (1) aus Symphytum aufweisend die folgenden Schritte:
- Bereitstellen von frischen, gefrorenen und/oder getrockneten Pflanzenteilen der Symphytum;
- Versetzen der Pflanzenteile mit einem niedermolekularem Alkohol, insbesondere mit Ethanol;
- Einstellen des Alkoholgehaltes der ersten Extraktionsphase (1) auf 65-75 Vol%, insbesondere auf 70 Vol%.

17. Verfahren zur Herstellung einer zweiten, schleimhaltigen Extraktionsphase (2) aus einer bereitgestellten ersten, im Wesentlichen schleimfreien Extraktionsphase (1) aus Symphytum, wobei die zweite, schleimhaltige Extraktionsphase (2) durch Mazeration, insbesondere durch Rührmazeration, der ersten Extraktionsphase (1) hergestellt wird, wobei der Alkoholgehalt der Extraktionsphase auf 45 Vol% - 55 Vol%, insbesondere auf 50 Vol% eingestellt wird.

18. Verfahren zum Fällen von Schleimpolysacchariden (SPS) aufweisend eine differentiellen Schleimfällung, wobei die differentielle Schleimfällung folgende Schritte aufweist:
- erste differentielle Schleimfällung von hochmolekularen Schleimpolysacchariden (SPS) als erster Niederschlag (N1) in einem Suspensionssystem (SS) aus Symphytum, Abtrennung des ersten Niederschlags (N1) und eines Extraktionsrückstands als Raffinat (R) und Gewinnung einer ersten Extraktphase (A);
- zweite differentielle Schleimfällung von niedermolekularen Schleimpolysacchariden (SPS) als zweiter Niederschlag (N2) aus der ersten Extraktphase (A), Abtrennung des zweiten Niederschlags (N2) und Gewinnung einer zweiten Extraktphase (B).

19. Verfahren zur selektive Entfernung von Pyrrolizidin-Alkaloiden (PA) von einem aus einem Suspensionssystem (SS) aus Symphytum gefällten und von einem Extrakt getrennten Niederschlag aufweisend die folgenden Schritte:
- Resuspendierung des gefällten und abgetrennten Niederschlags in einer Waschlösung, insbesondere in einer Waschlösung aufweisend einen sauren, niedermolekularen Alkohol, wobei der Alkohol insbesondere einen pH-Wert von 2-4 aufweist, und
Abtrennen des gewaschenen Niederschlags von der Waschlösung;
oder
- Resuspendierung des gefällten und abgetrennten Niederschlags in hochprozentigem, niedermolekularem Alkohol, insbesondere Ethanol, Versetzen des resuspendierten Niederschlags mit einem Katalysator insbesondere einem sauren Katalysator auf gelförmiger Polymerbasis, wobei der Katalysator insbesondere Sulfonsäuregruppen auf der Polymeroberfläche aufweist, und
Abtrennen des Katalysators vom resuspendierten Niederschlag und anschliessender Rückgewinnung des gereinigten Niederschlags aus der Suspension;
wobei das Resuspendieren und Reinigen mit dem gereinigten Niederschlag wiederholbar ist; und
wobei der gereinigte Niederschlag insbesondere maximal 1 ppm Pyrrolizidin-Alkaloide (PA) aufweist.

20. Verfahren zur selektive Entfernung von Pyrrolizidin-Alkaloiden (PA) aus einem von einem Niederschlag in einem Suspensionssystem (SS) aus Symphytum getrennten Extraktphase, aufweisend mindestens einen der folgenden Schritte:
- Reinigung der Extraktphase mit Hilfe einer stationären Phase, wobei die stationäre Phase insbesondere einen Kationenaustauscher in saurer Form aufweist;
- Einstellen des pH-Wertes der gereinigten Extraktphase, auf 5.5-7, insbesondere auf 6.2-6.5; und
- Aufkonzentrierung der gereinigten Extraktphase, insbesondere mittels Destillation
wobei die gereinigte Extraktphase (B), maximal 0.5 ppm Pyrrolizidin-Alkaloide (PA), insbesondere maximal 0.1 ppm Pyrrolizidin-Alkaloide (PA) aufweist.
